# EUROPÄISCHE PATENTSCHRIFT

(11) **EP 3 750 865 B1**
(45) Veröffentlichungstag und Bekanntmachung des Hinweises auf die Patenterteilung: **30.03.2022**
(21) Anmeldenummer: 20178665.4
(22) Anmeldetag: 08.06.2020
(51) Int. Cl.: C07C 51/09, C07C 67/38, C07C 67/56, C07C 69/22, C07C 69/24, C07C 69/612, C07C 53/122, C07C 53/124, C07C 53/126, C07C 57/30

(54) **VERFAHREN ZUR HERSTELLUNG VON CARBONSÄUREN ODER DEREN SALZEN AUS KOHLENWASSERSTOFFEN**
METHOD FOR THE PREPARATION OF CARBOXYLIC ACIDS OR THEIR SALTS FROM HYDROCARBONS
PROCÉDÉ DE FABRICATION D'ACIDES CARBOXYLIQUES OU DE LEURS SELS À PARTIR D'HYDROCARBURES

(30) Priorität: 12.06.2019 EP 19179571
(43) Veröffentlichungstag der Anmeldung: 16.12.2020
(73) Patentinhaber: Evonik Operations GmbH, 45128 Essen (DE)
(72) Erfinder: Kucmierczyk, Peter, 44652 Herne (DE); Franke, Robert, 45772 Marl (DE); Fridag, Dirk, 45721 Haltern am See (DE); Knossalla, Johannes, 46514 Schermbeck (DE); Schäpertöns, Marc, 45657 Recklinghausen (DE); Gluth, Frederik, 45472 Mülheim an der Ruhr (DE)
(74) Vertreter: Evonik Patent Association

(56) Entgegenhaltungen:
- WO-A1-2013/107902
- US-A1- 2006 128 985
- PATRIZIA MARCHETTI ET AL: "Molecular Separation with Organic Solvent Nanofiltration: A Critical Review", CHEMICAL REVIEWS, Bd. 114, Nr. 21, 21. Oktober 2014 (2014-10-21), Seiten 10735-10806, XP055618006, US ISSN: 0009-2665, DOI: 10.1021/cr500006j

## Beschreibung

Die vorliegende Erfindung betrifft ein Verfahren zur Herstellung von Carbonsäuren oder deren Salzen durch Hydrolyse oder Verseifung eines Esters, der durch Alkoxycarbonylierung eines C2-bis C20-Kohlenwasserstoffs mit mindestens einer Mehrfachbindung, vorzugsweise mit mindestens einer olefinischen Doppelbindung, erhalten wird, bei dem das eingesetzte homogene Katalysatorsystem mittels Membrantrennung aus dem Produktgemisch abgetrennt wird. In einer Weiterbildung der vorliegenden Erfindung wird der so gebildete Ester durch Umesterung zu einem anderen Ester umgesetzt und dann hydrolysiert oder verseift.

Die Herstellung von Alkoholen in der großindustriellen Chemie erfolgt zum größten Teil über die Hydroformylierung zur Herstellung eines Aldehyds mit nachfolgender Oxidation des Aldehyds zur Carbonsäure. Wenngleich die Herstellung von Carbonsäuren im Wege der Hydroformylierung mit nachfolgender Oxidation ein seit Jahrzehnten industriell etablierter und bewährter Prozess ist, weist er immer noch Verbesserungspotential auf (vgl. DE 100 10 771 C1 oder EP 1 854 778 A1). Ein Problem bei dieser Syntheseroute ist, dass bei der Hydroformylierung Übergangsmetall-haltige Katalysatorsysteme eingesetzt werden, die üblicherweise teuer sind oder kostenintensiv hergestellt werden müssen. Ein weiteres Problem ist die Bildung von Nebenprodukten, die sich selbst dann zu beobachten ist, wenn kein Katalysator eingesetzt wird und/oder die Reaktionsbedingungen vergleichsweise milde sind. Ein weiteres Problem ist der erhöhte Bedarf an Sicherheitsausrüstung bei der Oxidation von Aldehyden, da in diesem Schritt Sauerstoff in reiner Form oder durch Luft der organischen Reaktionsmischung zugeführt wird. Die dadurch erzeugte explosive Atmosphäre muss gesondert und erschwert überwacht und geregelt werden. Dokument WO2004050599 offenbart die Herstellung von Vinylacetatverbindungen durch Carbonylierung von Vinylacetatverbindungen.

Zur Vermeidung von Nebenreaktionen und zur Erhöhung der Selektivität ist in der Literatur vorgeschlagen worden Alkali- und/oder Erdalkalimetallverbindungen zur Reaktionslösung zu geben. Nachteil dieser Variante ist aber, dass diese Verbindungen eine inhibierende Wirkung auf die Oxidation der Aldehyde haben und deshalb längere Reaktionszeiten notwendig sind, um ausreichende Umsätze zu erzielen.

Der vorliegenden Erfindung lag daher die Aufgabe zu Grunde, einen alternativen Syntheseweg für die Herstellung von Carbonsäuren oder deren Salzen anzugeben, mit der sich die gewünschten Carbonsäuren bzw. deren Salze relativ einfach erschließen lassen und bei der auf die Anwesenheit von Alkali- und/oder Erdalkalimetallverbindungen verzichtet werden kann. Eine weitere wichtige Zielsetzung ist, dass das Herstellungsverfahren im großindustriellen Maßstab durchführbar ist. Es gilt die klassische Synthese durch eine andere Synthesetechnologie zu ersetzen, welche die Produkte des bekannten Verfahrens in besserer Qualität liefert. Darüber hinaus soll der neu zu schaffende Syntheseweg weniger unerwünschte Nebenprodukte bilden. Darüber hinaus soll zusätzliche Sicherheitsausrüstung, welche durch Anwesenheit von Sauerstoff notwendig ist, reduziert werden.

Gelöst wird diese Aufgabe durch ein zweistufiges Verfahren in welchem in einem ersten Schritt in einer Alkoxycarbonylierung ein Kohlenwasserstoff mit mindestens einer Mehrfachbindung mit Kohlenmonoxid und einem Alkohol zu einem Ester umgesetzt wird und in welchem in einem zweiten Schritt in einer Hydrolyse oder Verseifung der Ester in Anwesenheit eines sauren Katalysators bzw. eines Verseifungsmittels zu der gewünschten Carbonsäure bzw. zu dem gewünschten Carbonsäuresalz umgesetzt wird. Dabei wird in dem zweiten Schritt der ursprünglich eingesetzte Alkohol teilweise wieder freigesetzt.

Erfindungsgemäß umfasst das Verfahren zur Herstellung einer Carbonsäure oder deren Salz die folgenden Schritte:
a) Herstellung eines Esters durch Umsetzen (Carbonylieren) eines C2- bis C20-Kohlenwasserstoffs mit mindestens einer Mehrfachbindung, vorzugsweise mit mindestens einer olefinischen Doppelbindung mit Kohlenmonoxid und mit einem Alkohol A in Gegenwart eines homogenen Katalysatorsystems in einer Reaktionszone unter Erhalt eines Produktgemisches;
b) Durchführen einer Membrantrennung zur Abtrennung des homogenen Katalysatorsystems aus dem Produktgemisch, wodurch das homogene Katalysatorsystem und nicht umgesetzter Kohlenwasserstoff und/oder nicht umgesetzter Alkohol A, vorzugsweise nicht umgesetzter Alkohol A im Retentat angereichert werden und der in Schritt a) gebildete Ester im Permeat angereichert wird, wobei als Membranmaterial ein OSN-fähiges (Organic Solvent Nanofiltration) Membranmaterial, welches zumindest eine trennaktive Schicht aufweist, eingesetzt wird,;
c) Abtrennen des in Schritt a) gebildeten Esters aus dem Permeat in mindestens einem Trennverfahrensschritt, ausgewählt aus thermischer Trennung, beispielsweise Destillation Extraktion, Kristallisation und Membrantrennung;
d) Hydrolyse oder Verseifen des in Schritt a) hergestellten Esters in Anwesenheit eines sauren Katalysators oder eines Verseifungsmittels unter Erhalt eines Reaktionsgemisches, welches zumindest die Carbonsäure oder deren Salz, den abgespaltenen Alkohol A, Wasser und nicht umgesetzte Ester umfasst;
e) Abtrennen der in Schritt d) gebildeten Carbonsäure oder deren Salz in mindestens einem Trennverfahrensschritt, ausgewählt aus thermischer Trennung, beispielsweise Destillation Extraktion, Kristallisation und Membrantrennung.

Die bei der Umsetzung in Schritt a) eingesetzten Kohlenwasserstoffe müssen mindestens eine Mehrfachbindung aufweisen. Bevorzugt sind Kohlenwasserstoffe mit mindestens einer olefinischen Doppelbindung, besonders bevorzugt sind Kohlenwasserstoffe mit einer olefinischen Doppelbindung. Grundsätzlich ist die Anzahl der Kohlenstoffatome von Verbindung, die mindestens eine Mehrfachbindung, vorzugsweise mindestens eine olefinische Doppelbindung aufweisen, nicht begrenzt. Technische relevant sind aber nur die Carbonylierung von C2- bis C20-Kohlenwasserstoffen mit mindestens einer Mehrfachbindung, vorzugsweise mindestens einer olefinischen Doppelbindung. In einer bevorzugten Ausführungsform der vorliegenden Erfindung können C3- bis C16-Kohlenwasserstoffe, besonders bevorzugt C3- bis C12-Kohlenwasserstoffe mit mindestens einer Mehrfachbindung, vorzugsweise mindestens einer olefinischen Doppelbindung eingesetzt werden. Darunter fallen insbesondere n-Alkene, iso-Alkene, Cycloalkene und aromatische Alkene mit 2 bis 20 Kohlenstoffatomen, vorzugsweise 3 bis 16 Kohlenstoffatomen, besonders bevorzugt 3 bis 12 Kohlenstoffatomen.

Die vorgenannt beschriebenen Kohlenwasserstoffe können zusätzlich zu der mindestens einen olefinischen Doppelbindungen eine oder mehrere weitere funktionelle Gruppen enthalten. Beispiele für geeignete funktionelle Gruppen sind Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl-, Amino-, Ether-, Thioether-, Aryl-, Heteroaryl- oder Silylgruppen und/oder Halogensubstituenten.

Besonders bevorzugte Kohlenwasserstoffe, die in Schritt a) des erfindungsgemäßen Verfahrens eingesetzt werden, weisen nur eine olefinische Doppelbindung auf, insbesondere n-Alkene und iso-Alkene mit 2 bis 20 Kohlenstoffatomen, vorzugsweise 3 bis 16 Kohlenstoffatomen, besonders bevorzugt 3 bis 12 Kohlenstoffatomen. Die eingesetzten Kohlenwasserstoffe sind vorzugsweise unsubtituiert.

Die eingesetzten und vorher beschriebenen Kohlenwasserstoffe werden erfindungsgemäß mit Kohlenmonoxid (CO) und einem Alkohol zum entsprechenden Ester in Schritt a) umgesetzt. Das Kohlenmonoxid kann dabei direkt als Einsatzgemisch oder durch die Zugabe eines Kohlenmonoxid-haltigen Gases, ausgewählt aus Synthesegas, Wassergas, Generatorgas und anderen Kohlenmonoxid-haltigen Gasen bereitgestellt werden. Möglich ist es auch, das Kohlenmonoxid dadurch bereitzustellen, dass das Kohlenmonoxid-haltige Gas vorher in einer für den Fachmann bekannten Weise in seine Komponenten aufgetrennt wird und das Kohlenmonoxid zur Reaktionszone geleitet wird. Das Kohlenmonoxid kann dabei weiterhin einen gewissen Anteil an Wasserstoff oder anderen Gasen enthalten, weil eine vollständige Auftrennung kaum zu realisieren ist.

Der bei der Umsetzung in Schritt a) eingesetzte Alkohol ist ein Mono- oder Polyalkohol (zwei oder mehr OH-Gruppen) mit 1 bis 50 Kohlenstoffatomen, vorzugsweise 1 bis 15 Kohlenstoffatomen, besonders bevorzugt 1 bis 10 Kohlenstoffatomen oder eine Mischung aus zwei oder mehr Mono- und/oder Polyalkoholen. In einer bevorzugten Ausführungsform ist der Polyalkohol ein Diol, Triol oder Tetrol, vorzugsweise ein Diol oder Triol mit der vorgenannten Anzahl an Kohlenstoffatomen. Geeignete Alkohole für die Umsetzung in Schritt a) sind Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol oder Mischungen daraus, vorzugsweise Ethanol, 1-Propanol, 1-Butanol, 1-Pentanol, 1-Hexanol, 2-Propanol, tert-Butanol, 3-Pentanol, 2-Propylheptanol.

Der in Schritt a) eingesetzte Alkohol wird vorzugsweise in einem Molverhältnis zum eingesetzten Kohlenwasserstoff (Alkohol: Kohlenwasserstoff) von 2 bis 20, weiterhin bevorzugt 3 bis 10 und besonders bevorzugt 4 bis 6 eingesetzt.. Der Monoalkohol wird somit - bezogen auf den eingesetzten Kohlenwasserstoff - im molaren Überschuss hinzugefügt. Dadurch kann der Alkohol sowohl als Edukt für die Carbonylierung als auch als Lösemittel fungieren. Der in Schritt a) eingesetzte Alkohol wird, wenn es sich um einen Polyalkohol handelt, in einem Molverhältnis zum eingesetzten Kohlenwasserstoff (Kohlenwasserstoff: Polyalkohol) von 2 bis 20, vorzugsweise von 3 bis 10 und besonders bevorzugt von 4 bis 8 eingesetzt. Der Polyalkohol wird bezogen auf den eingesetzten Kohlenwasserstoff also im molaren Unterschuss hinzugefügt.

Die erfindungsgemäße Umsetzung in Schritt a) wird in Gegenwart eines homogenen Katalysatorsystems durchgeführt. Bevorzugt umfasst das homogene Katalysatorsystem zumindest ein Metall aus der Gruppe 8 bis 10 des Periodensystems der Elemente (PSE) oder eine Verbindung davon, einen phosphorhaltigen Liganden und eine Säure als Co-Katalysator.

Das Metall aus der Gruppe 8 bis 10 des PSE ist vorzugsweise Palladium. Das Palladium wird bevorzugt in Form einer Vorläuferverbindung als Palladiumverbindung eingesetzt, die durch den phosphorhaltigen Liganden koordiniert wird. Beispiele für Palladiumverbindungen, die als Vorläuferverbindungen eingesetzt werden können, sind Palladiumchlorid [PdCl₂], Palladium(II)-Acetylacetonat [Pd(acac)₂], Palladium(II)-Acetat [Pd(OAc)₂], Dichloro-(1,5-cyclooctadien)palladium(II) [Pd(cod)Cl₂], Bis(dibenzylideneaceton)palladium(0) [Pd(dba)₂], Tris(dibenzylideneaceton)dipalladium(0) [Pd2(dba)₃] Bis(acetonitril)-dichloropalladium(II) [Pd(CH₃CN)₂Cl₂], Palladium(cinnamyl)-dichlorid [Pd(cinnamyl)Cl₂]. Vorzugsweise kommen die Verbindungen [Pd(acac)₂] oder [Pd(OAc)₂] zum Einsatz. Die Metallkonzentration von Palladium in Schritt a) beträgt vorzugsweise zwischen 0,01 und 0,6 Mol-%, bevorzugt zwischen 0,03 und 0,3 Mol-%, besonders bevorzugt zwischen 0,04 und 0,2 Mol-% bezogen auf die Stoffmenge des eingesetzten Kohlenwasserstoffs.

Geeignete phosphorhaltige Liganden des erfindungsgemäßen Katalysatorsystems weisen vorzugsweise eine Bidentat-Struktur auf. Bevorzugte phosphorhaltige Liganden für die das erfindungsgemäße Katalysatorsystem sind benzolbasierte Diphosphinverbindungen, wie sie beispielsweise in der EP 3 121 184 A2 offenbart worden sind. Die Liganden können in einer Vorreaktion mit dem Palladium kombiniert werden, sodass der Palladium-Ligand-Komplex zur Reaktionszone geführt wird, oder in situ zur Reaktion gegeben und dort mit dem Palladium kombiniert werden. Das Molverhältnis von Ligand : Metall kann für die beschriebene Umsetzung in Schritt a) 1 : 1 bis 10 : 1, vorzugsweise 2 : 1 bis 6 : 1, besonders bevorzugt 3 : 1 bis 5 : 1 betragen.

Das homogene Katalysatorsystem umfasst weiterhin eine Säure, wobei es sich insbesondere um eine Brönsted- oder eine Lewis-Säure handelt. Als Lewis-Säure können insbesondere Lewis Säuren mit einem LAU-Wert von mehr als 25, vorzugsweise mit einem LAU-Wert von 29. Der LAU-Wert ist eine Methode zur Bestimmung der Stärke von Lewis-Säuren (JR Gaffen et al., Chem, Vol. 5, Issue 6, S. 1567-1583). Als Lewis-Säure werden vorzugsweise Aluminiumtriflat, Aluminiumchlorid, Aluminiumhydrid, Trimethylaluminium, Tris(pentafluorophenyl)boran, Bortrifluorid, Bortrichlorid oder Mischungen daraus eingesetzt werden. Von den genannten Lewis-Säuren wird bevorzugt Aluminiumtriflat eingesetzt. Die Lewis-Säure wird vorzugsweise in einem Molverhältnis Lewis-Säure : Ligand von 1 : 1 bis 20 : 1, vorzugsweise 2 : 1 bis 15 : 1, besonders bevorzugt 5 : 1 bis 10 : 1 hinzugegeben.

Geeignete Brönsted-Säuren haben vorzugsweise eine Säurestärke von pKs ≤ 5, besonders bevorzugt eine Säurestärke von pKs ≤ 3. Die angegebene Säurestärke pKs bezieht sich auf den bei Normalbedingungen (25°C, 1,01325 bar) bestimmten pKs-Wert. Bei einer mehrprotonigen Säure bezieht sich die Säurestärke pKs im Rahmen dieser Erfindung auf den pKs-Wert des ersten Protolyseschrittes. Die Brönsted-Säure wird vorzugsweise in einem Molverhältnis Brönsted-Säure : Ligand von 1 : 1 bis 15 : 1, vorzugsweise 2 : 1 bis 10 : 1, besonders bevorzugt 3 : 1 bis 4 : 1 hinzugegeben.

Als Brönsted-Säuren können insbesondere Perchlorsäure, Schwefelsäure, Phosphorsäure, Methylphosphonsäure oder Sulfonsäuren eingesetzt werden. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure. Vorzugsweise handelt es sich bei der Säure um Schwefelsäure.

Die Umsetzung bzw. Carbonylierung des eingesetzten Kohlenwasserstoffs mit olefinischer Doppelbindung in Schritt a) wird vorzugsweise bei einer Temperatur von 25 bis 140 °C, weiterhin bevorzugt bei einer Temperatur von 80 bis 130 °C und besonders bevorzugt bei einer Temperatur von 90 bis 120 °C durchgeführt. Der Druck in Schritt a) kann zwischen 5 und 60 bar, vorzugsweise zwischen 10 und 40 bar, besonders bevorzugt zwischen 15 und 30 bar betragen.

Die beschriebene Umsetzung in Schritt a) findet in einer geeigneten Reaktionszone statt. Die Reaktionszone für die Umsetzung umfasst mindestens einen Reaktor, kann aber auch aus zwei oder mehr Reaktoren bestehen. Der mindestens eine Reaktor kann insbesondere aus der Gruppe, bestehend aus einem Rührkesselreaktor, einem Schlaufenreaktor, einem Jet-Loop-Reaktor, einem Blasensäulenreaktor oder Kombinationen daraus, ausgewählt sein. Sind mehrere Reaktoren vorhanden können die Reaktoren gleich oder unterschiedlich sein.

Durch die oben beschriebene Umsetzung in Schritt a) wird ein flüssiges Produktgemisch erhalten, die zumindest den durch die Umsetzung gebildeten Ester, das homogene Katalysatorsystem, nicht umgesetzte Alkohole A, und ggf. weitere Komponenten wie Leichtsieder, beispielsweise leichtsiedende Nebenprodukte wie Ether, und/oder Hochsieder und/oder nicht umgesetzte Kohlenwasserstoffe umfasst. Das Produktgemisch wird dann der nachfolgenden Membrantrennung in Schritt b) zugeführt. Bei der Umsetzung in Schritt a) kann zudem ein Abgas, welches zumindest aus unreaktiven Verunreinigungen wie Stickstoff, Wasserstoff und leichtsiedenden Nebenprodukten (beispielsweise die bereits genannten Ether) aus der Reaktionszone entnommen werden. Die Verunreinigungen und leichtsiedenden Nebenprodukte könnten sich akkumulieren und den Partialdruck des Reaktionsgases (CO) auf lange Sicht erniedrigen, wodurch die Reaktion verlangsamt werden könnte.

Im nachfolgenden Schritt b) wird das Produktgemisch einer Membrantrennung zugeführt, um das homogene Katalysatorsystem vom Produktgemisch abzutrennen. Durch das erfindungsgemäße Membranmaterial werden das homogene Katalysatorsystem und nicht umgesetzter Kohlenwasserstoff und/oder nicht umgesetzter Alkohol im Retentat angereichert, während der in Schritt a) gebildete Ester im Permeat angereichert wird. Das Permeat, welches den gebildeten Ester enthält, wird dann zum nachfolgenden Schritt c) geführt. Das Retentat, welches das angereicherte homogene Katalysatorsystem umfasst, wird dann in die Reaktionszone zurückgeführt. Bei der Rückführung des Retentats kann weiterhin ein Purgestrom, welcher inerte Alkane, leichtsiedende Nebenprodukte (bspw. Ether), mögliche Abbauprodukte des Katalysatorsystems oder andere Verunreinigungen enthalten kann, die durch die eingesetzten Kohlenwasserstoffströme eingetragen werden, wie beispielsweise Spuren von Wasser oder Stickstoff, entnommen werden, um eine Akkumulation in der oder den Reaktionszonen zu vermeiden. Die Rückführung des Retentats sorgt dafür, dass das bei der Membrantrennung im Retentat anfallende Katalysatorsystem zur Reaktion zurückgeführt wird. Dadurch werden Katalysatorverluste durch Abscheidung oder Desaktivierung minimiert und das Verfahren kostengünstiger gestaltet. Katalysatorverluste sind meistens nicht ganz zu vermeiden, aber die erwähnte Verringerung der Verluste führt dazu, dass weniger Katalysator durch Zuführung von frischem Katalysator ersetzt werden muss.

Die Membrantrennung beruht auf der Semipermeabilität des Membranmaterials, welches für bestimmte Stoffe durchlässig und für andere Stoffe undurchlässig ist. Das in Schritt b) des erfindungsgemäßen Verfahrens eingesetzte Membranmaterial ist ein OSN-Membranmaterial (OSN = Organic Solvent Nanofiltration). Ein solches Membranmaterial besteht vorzugsweise zumindest aus einer eher dünnen trennaktiven Schicht (auch: aktiven Trennschicht) und optional einem dickeren Backing, worauf sich die trennaktive Schicht befindet. Bevorzugt besteht das erfindungsgemäße Membranmaterial zumindest aus einer trennaktiven Schicht und einem Backing. Zwischen trennaktiver Schicht und Backing können eine oder mehrere Zwischenschichten vorhanden sein. In einer bevorzugten Ausführungsform besteht das Membranmaterial nur aus der trennaktiven Schicht und dem Backing. Das Membranmaterial, aus zumindest trennaktiver Schicht und Backing, ist vorzugsweise säurestabil sein, damit das Membranmaterial nicht durch die im Produktgemisch als Co-Katalysator befindliche Säure beschädigt wird. Der Begriff "säurestabil" meint im Rahmen der vorliegenden Erfindung, dass das Membranmaterial mindestens 300 h in Anwesenheit der Säure des Katalysatorsystems, insbesondere einer Brönsted-Säure mit einem pKs ≤ 5, besonders bevorzugt mit einem pKs ≤ 3 oder einer Lewis-Säure mit einem LAU-Wert von mehr als 25, vorzugsweise mit einem LAU-Wert von 29, stabil ist und nicht zerstört wird, wodurch die eigentliche Trennwirkung nicht mehr erzielt werden könnte.

Das Backing weist insbesondere eine poröse Struktur auf, die für das durch die trennaktive Schicht gelangte Permeat durchlässig ist. Das Backing hat eine stabilisierende Funktion und dient als Träger für die trennaktive Schicht. Das Backing kann grundsätzlich aus jedem geeigneten porösen Material bestehen. Voraussetzung ist jedoch, dass das Material säurestabil ist. Das Backing kann auch aus dem gleichen Material bestehen wie die trennaktive Schicht.

Die erfindungsgemäße trennaktive Schicht besteht vorzugsweise aus einem PAEK-Polymer (Polyaryletherketon). PAEK zeichnet sich dadurch aus, dass innerhalb der Widerholungseinheit Arylgruppen abwechselnd über eine Etherfunktionalität und eine Ketonfunktionalität verknüpft sind. Eine erfindungsgemäß bevorzugte trennaktive Schicht besteht aus PEEK (Polyetheretherketon). Als trennaktive Schicht können insbesondere bevorzugt PEEK-Polymere mit einem Sulfonierungsgrad von weniger als 20%, besonders bevorzugt mit einem Sulfonierungsgrad von weniger als 10% eingesetzt werden. Die entsprechenden PEEK-Polymere und deren Herstellung sind in der WO 2015/110843 A1 beschrieben. Es hat sich überraschend gezeigt, dass dieses Material besonders stabil ist, insbesondere auch gegenüber der Säure als Co-Katalysator des homogenen Katalysatorsystems. Außerdem zeichnet sich das erfindungsgemäße PEEK-Material dadurch aus, dass es als trennaktive Schicht eingesetzt bevorzugt die gebildeten Ester durchlässt, während selbst die eingesetzten Eduktalkohole zumindest teilweise zurückgehalten werden und sich dadurch im Retentat anreichern. Dadurch kann die nachfolgende Aufarbeitung des restlichen Produktgemisches wirtschaftlicher und nachhaltiger betrieben werden, weil verglichen mit bekannten Membranmaterialien weniger Alkohole abgetrennt werden müssen.

Die Membrantrennung in Schritt b) wird vorzugsweise Temperatur von 25 bis 100°C, weiterhin bevorzugt 30 bis 80°C und besonders bevorzugt 40 bis 70°C durchgeführt. Um das Produktgemisch auf die bei der Membrantrennung bevorzugte vorherrschende Temperatur zu bringen, kann das Produktgemisch gekühlt werden. Neben einer aktiven Kühlung unter Verwendung eines Kühlmediums, kann die Kühlung auch über einen Wärmetauscher erreicht werden, wodurch ein anderer Strom innerhalb des erfindungsgemäßen Verfahrens erhitzt wird. Optional ist auch ein Degasingschritt zwischen der Reaktionszone in Schritt a) und der Membrantrennung in Schritt b) vorhanden, um vorher leicht flüchtige Verbindungen wie Kohlenmonoxid und/oder restliche, über das Abgas nicht abgetrennte unreaktiven Verunreinigungen wie Stickstoff, Wasserstoff, Alkane und leichtsiedenden Nebenprodukte (beispielsweise die bereits genannten Ether) aus der Produktmischung zu entfernen. Dabei wird das Produktgemisch erst unter den Partialdruck der gelösten Komponenten wie Kohlenmonoxid entspannt, so dass diese ausgasen, um dann wieder den Druck zu erhöhen, der in der Membrantrennung vorgesehen ist.

Der Transmembrandruck (TMP) kann in Schritt b) 10 bis 60 bar, vorzugsweise 15 bis 55 bar, besonders bevorzugt 20 bis 50 bar betragen (relativ). Der permeatseitige Druck kann dabei oberhalb des atmosphärischen Drucks bis 15 bar, vorzugsweise 3 bis 7 bar betragen, woraus sich anhand des TMP dann der retentaseitige Druck ergibt. In einer bevorzugten Ausführungsform sollte bei den Druckverhältnissen und insbesondere beim permeatseitigen Druck darauf geachtet werden, dass der Druck in Abhängigkeit von dem eingesetzten Kohlenwasserstoff, dem eingesetzten Alkohol und der vorhandenen Temperatur eingestellt wird, um eine Verdampfung nach dem Durchtritt durch die Membran zu vermeiden, da dadurch die gesamte Fahrweise instabil werden könnte. Gleiches gilt grundsätzlich auch für gelöste Komponenten wie Kohlenmonoxid, die optional durch den bereits genannten Degasingschritt entfernt werden können.

Das Permeat aus der Membrantrennung (Schritt b)) wird im nachfolgenden Schritt c) einem Trennverfahren ausgewählt aus der Gruppe, bestehend aus einer thermischen Trennung, beispielsweise Destillation, einer Extraktion, einer Kristallisation oder einer weiteren Membrantrennung, unterworfen, um den in Schritt a) gebildeten Ester vom restlichen Permeat abzutrennen. Das Trennverfahren ist vorzugsweise eine Destillation. Die entsprechenden Verfahrensbedingungen sind dem Fachmann bekannt.

Es kann vorkommen, dass bei dem in Schritt c) verwendeten Trennverfahren, insbesondere bei der Destillation, nicht nur der gebildete Ester aus dem Permeat abgetrennt wird, sondern auch die möglicherweise entstandenen Hochsieder, beispielsweise hochsiedende Nebenprodukte, die bei der Umsetzung in Schritt a) anfallen können. Um diese Hochsieder zu entfernen kann das erfindungsgemäße Verfahren einen Aufreinigungsschritt aufweisen, nämlich die Aufreinigung des gebildeten Esters durch Abtrennung des Esters von im Permeat enthaltenden Hochsiedern mittels thermischer Trennung, Extraktion, Kristallisation oder Membrantrennung. Bevorzugt wird ein thermisches Trennverfahren, besonders bevorzugt eine weitere Destillation eingesetzt, um die gebildeten Ester aufzureinigen. Die Verfahrensbedingungen sind dem Fachmann bekannt.

Das in Schritt c) erhaltene zu großen Teilen vom in Schritt a) gebildeten Ester befreite Permeat, welches mindestens nicht umgesetzte Alkohole und/oder nicht umgesetzte Kohlenwasserstoffe enthält, wird in einer bevorzugten Ausführungsform einer Recyclekomponentenabtrennung unterworfen. Dabei werden die nicht umgesetzten Alkohole und/oder nicht umgesetzten Kohlenwasserstoffe vom restlichen Permeat, insbesondere den dort enthaltenen Leichtsiedern, mittels thermischer Trennung, Extraktion, Kristallisation oder Membrantrennung abgetrennt. Bevorzugt wird ein thermisches Trennverfahren, besonders bevorzugt eine weitere Destillation eingesetzt, um die nicht umgesetzten Alkohole und/oder nicht umgesetzten Kohlenwasserstoffe vom restlichen Permeat abzutrennen. Die Verfahrensbedingungen sind dem Fachmann bekannt. Die dabei erhaltenen nicht umgesetzten Alkohole und/oder die nicht umgesetzten Kohlenwasserstoffe können dann in die Reaktionszone zurückgeführt werden.

Der nach dem erfindungsgemäßen Verfahren gebildete Ester kann in zwei weiteren Verfahrensschritten c1) und c2) umgeestert werden. Dabei wird der Teil des Esters, der dem in Schritt a) eingesetzten ersten Alkohol A entspricht, durch einen zweiten Alkohol B ausgetauscht. Diese Umesterung wird nach dem oben genannten Schritt c), optional nach dem möglichen Aufreinigungsschritt, durchgeführt und umfasst die folgenden Schritte:
c1) Umestern des in Schritt a) gebildeten Esters mit einem zweiten Alkohol B, wobei dieser zweite Alkohol sich von dem im Schritt a) eingesetzten Alkohol A unterscheidet, in einer zweiten Reaktionszone unter Erhalt eines zweiten Produktgemischs, welches zumindest den Ester mit dem zweiten Alkohol B, den abgespaltenen ersten Alkohol A und nicht umgesetzten zweiten Alkohol B umfasst;
c2) Abtrennen des gebildeten Esters mit dem zweiten Alkohol B vom restlichen zweiten Produktgemisch und insbesondere vom abgespaltenen ersten Alkohol A durch thermische Trennung und/oder mittels Membrantrennung und Rückführung des abgespaltenen ersten Alkohols A in die Reaktionszone aus Schritt a), sowie Rückführung des nicht umgesetzten Alkohols B in die zweite Reaktionszone.

In Schritt c1) erfolgt die eigentliche Umeesterung, also die Abspaltung des eigentlich in Schritt a) angebundenen ersten Alkohols A und die Anbindung des zweiten Alkohols B. Dazu wird der in Schritt a) gebildete Ester in einer Reaktionszone mit einem zweiten Alkohol B, der sich vom ersten Alkohol A unterscheidet, umgesetzt. In einer besonders bevorzugten Ausführungsform ist der bei der Umesterung eingesetzte zweite Alkohol B im Vergleich zu dem in Schritt a) eingesetzten ersten Alkohol A ein höhersiedender Alkohol. Der zweite Alkohol B wird bei der Umesterung vorzugsweise im Überschuss zugegeben, um die Umesterungsreaktion zu begünstigen.

Der bei der Umesterung in Schritt c1) eingesetzte zweite Alkohol ist vorzugsweise ein Mono- oder Polyalkohol (zwei oder mehr OH-Gruppen) mit 1 bis 50 Kohlenstoffatomen, weiterhin bevorzugt mit 1 bis 15 Kohlenstoffatomen, besonders bevorzugt mit 1 bis 10 Kohlenstoffatomen oder eine Mischung aus zwei oder mehr Mono- und/oder Polyalkoholen, mit der Maßgabe, dass der in Schritt a) eingesetzte erste Alkohol A und der zweite Alkohol B nicht identisch sind. In einer bevorzugten Ausführungsform ist der Polyalkohol ein Diol, Triol oder Tetrol, vorzugsweise ein Diol oder Triol mit der vorgenannten Anzahl an Kohlenstoffatomen. Geeignete Alkohole für die Umsetzung in Schritt a) sind Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol, Pentaerythrit, Neopentylglykol, Trimethylolpropan, Dipentaerythritol oder Mischungen daraus, vorzugsweise Ethanol, 1-Propanol, 1-Butanol, 1-Pentanol, 1-Hexanol, 2-Propanol, tert-Butanol, 3-Pentanol, 2-Propylheptanol.

Die Umesterung in Schritt c1) wird vorzugsweise säure- oder basenkatalysiert durchgeführt. Als Säuren können Brönsted- oder Lewis-Säuren eingesetzt werden.

Geeignete Brönsted-Säuren für die Umesterung in Schritt c1) sind Perchlorsäure, Schwefelsäure, Phosphorsäure, Methylphosphonsäure oder eine Sulfonsäure, beispielsweise Methansulfonsäure, Trifluormethansulfonsäure, tert-Butansulfonsäure, p-Toluolsulfonsäure(pTSA), 2-Hydroxypropan-2-sulfonsäure, 2,4,6-Trimethylbenzolsulfonsäure oder Dodecylsulfonsäure. Vorzugsweise werden Schwefelsäure oder eine Sulfonsäure als Brönsted-Säure eingesetzt, besonders bevorzugt Schwefelsäure. Es können auch Metall oder deren Verbindungen eingesetzt werden, beispielsweise Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat sowie Natriummethanolat und Kaliummethanolat.

Geeignete Lewis-Säuren für die Umesterung in Schritt c1) sind Titan(IV)-isopropoxid, Bu₂SnO, BuSn(O)OH oder Aluminiumtriflat. Bevorzugt eingesetzte Lewis Säuren sind Titan(IV)-isopropoxid, und Aluminiumtriflat

Geeignete Basen für die Umesterung in Schritt c1) sind Alkalimetalle, Alkalialkoxide, Alkali- oder Erdalkaliacetate oder -oxide, Alkali- oder Erdalkalialkoholate, wie NaEtOH oder MgEtOH oder Alkalicarbonate, wie K₂CO₃ oder Cs₂CO₃. Es können aber auch basische Ionenaustauscher oder NaOH eingesetzt werden. Bevorzugt werden Na- oder Mg-Alkoholate, wie NaEtOH oder MgEtOH, eingesetzt.

Die säurekatalysierten Umesterung wird vorzugsweise bei einer Temperatur von 60 bis 220 °C, weiterhin bevorzugt von 100 bis 210 °C und besonders bevorzugt bei 130 bis 200 °C durchgeführt. Die Reaktion findet vorzugsweise oberhalb des Siedepunktes des abzuspaltenden ersten Alkohols A statt, um den abgespaltenen ersten Alkohol A direkt aus dem Reaktionsgemisch zu entfernen und somit eine Gleichgewichtsverlagerung zur Produktseite zu begünstigen. Der zweite Alkohol B wird dabei vorzugsweise in einem signifikanten Überschuss, beispielsweise 30 : 1, zum in Schritt a) gebildeten Ester hinzugegeben.

Die basenkatalysierte Umesterung findet vorzugsweise bei einer Temperatur von 20 bis 100 °C statt.

Durch die beschriebe Umesterung wird ein zweites Produktgemisch erhalten, welches zumindest den Ester mit dem zweiten Alkohol B, den abgespaltenen ersten Alkohol A und nicht umgesetzte zweite Alkohole B umfasst.

Der in Schritt c1) gebildete zweite Ester wird im nachfolgenden Schritt c2) vom restlichen zweiten Produktgemisch abgetrennt. Die Abtrennung wird mittels thermischer Trennung, vorzugsweise Destillation, und/oder mittels Membrantrennung, insbesondere mit den oben beschriebenen Membranmaterialien, durchgeführt. Die entsprechenden Verfahrensbedingungen sind dem Fachmann bekannt.

Es kann vorkommen, dass bei dem in Schritt c2) verwendeten Trennverfahren, insbesondere bei der Destillation, nicht nur der gebildete Ester vom restlichen zweiten Produktgemisch abgetrennt wird, sondern auch möglicherweise gebildete Hochsieder, beispielsweise hochsiedende Nebenprodukte, die bei der Umsetzung in Schritt c1) anfallen können. Um diese Hochsieder zu entfernen kann das erfindungsgemäße Verfahren einen Aufreinigungsschritt aufweisen, nämlich die Aufreinigung des in Schritt c1) gebildeten Esters durch Abtrennung des Esters von den vorhandenen Hochsiedern mittels thermischer Trennung, Extraktion, Kristallisation oder Membrantrennung. Bevorzugt wird ein thermisches Trennverfahren, besonders bevorzugt eine weitere Destillation eingesetzt, um die gebildeten Ester aufzureinigen. Die Verfahrensbedingungen sind dem Fachmann bekannt.

Der in Schritt a) hergestellte und in Schritt c) aus dem Permeat abgetrennte und ggf. aufgereinigte Ester wird dann in Schritt d) einer Hydrolyse oder Verseifung unterworfen. Durch den dabei eingesetzten sauren Katalysator bzw. das dabei eingesetzte Verseifungsmittel wird die Estergruppe gespalten, wodurch eine Carbonsäure oder ein Carbonsäuresalz entsteht und der bei der Esterbildung in Schritt a) angebundenen Alkohol A bzw. den bei der Umesterung angebundenen Alkohol B zurückgewonnen werden kann. Es entsteht demnach bei der Verseifung ein Reaktionsgemisch, welches zumindest die Carbonsäure oder deren Salz, den Alkohol A oder B und nicht umgesetzte Ester umfasst.

Ohne Umesterung kann der mit der Hydrolyse oder Verseifung in Schritt d) zurückgewonnen Alkohol A in einem nachfolgenden Prozessschritt aus dem entstandenen Alkoholgemisch abgetrennt und in die erste Reaktionszone zurückgeführt werden. Wurde eine Umesterung durchgeführt und bei der Hydrolyse oder Verseifung in Schritt d) der Alkohol B zurückgewonnen, kann dieser Alkohol B im nachfolgenden Prozessschritt aus dem entstandenen Reaktionsgemisch abgetrennt und in die zweite Reaktionszone zurückgeführt werden.

Die Hydrolyse ist eine bekannte chemische Reaktion, bei der ein Ester mit Hilfe eines sauren Katalysators in eine Carbonsäure unter Abspaltung eines Alkohols überführt wird. Die typischen Bedingungen bei der Hydrolyse sind dem Fachmann bekannt.

Die Hydrolyse in Schritt d) findet in Anwesenheit eines sauren heterogenen oder homogenen Katalysators statt. Bekannte homogene Katalysatoren sind saure Verbindungen wie zum Beispiel Brönsted-Säuren, insbesondere HCl, H₂SO₄, Phosphorsäure, p-Toluolsulfonsäure und 4-Dodecylbenzolsulfonsäure, oder Lewis Säuren, insbesondere AlCl₃, ZnCl2, HfCl₄·2THF, Al(OTf)₃. Bekannte heterogene Katalysatoren sind saure Verbindungen wie zum Beispiel Kationenaustauscher, saure H-Zeolithe, saure funktionalisierte Metalloxide mit Silica und Heteropolysäuren. Geeignete heterogene Katalysatoren sind insbesondere Amberlyst^{®} 15, Amberlyte^{®} IR 120 (H), HClO₄-SiO₂, 3-Propylsulfonsäure funktionalisierte Silica, Nafion^{®}-SiO₂, Aciplex^{®}-SiO₂, Cs_{2.5}H_{0.5}PW₁₂O₄₀, H-ZSM5, H-ZSM-5-C18, Z-beta-H-25, Z-beta-H-38, Z-beta-H-150, Z-beta-H-360, Z-Y-H-60, Z-Y-H-80.

Das Reaktionsgemisch aus der Hydrolyse in Schritt d) wird im nachfolgenden Schritt e) in mindestens einem Trennverfahrensschritt ausgewählt aus der Gruppe, bestehend aus einer thermischen Trennung, beispielsweise Destillation, einer Extraktion, einer Kristallisation oder einer weiteren Membrantrennung, unterworfen, um die in Schritt d) gebildete Carbonsäure oder deren Salz vom restlichen Reaktionsgemisch abzutrennen. Das Trennverfahren ist vorzugsweise eine Destillation. Die entsprechenden Verfahrensbedingungen sind dem Fachmann bekannt. Es kann auch eine mehrstufige Destillation durchgeführt werden.

Die Verseifung ist eine bekannte chemische Reaktion, bei der ein Ester mit Hilfe eines basischen oder enzymatischen Verseifungsmittels in ein Carbonsäuresalz unter Abspaltung eines Alkohols überführt wird. Die typischen Bedingungen bei der Verseifung sind dem Fachmann bekannt.

Die Verseifung in Schritt d) findet in Anwesenheit eines Verseifungsmittels statt. Bekannte Verseifungsmittel sind basische Verbindungen wie Kaliumhydroxid, Kalium(hydrogen)carbonat, Natriumhydroxid, Natrium(hydrogen)carbonat oder Aminverbindungen. Möglich ist auch eine Verseifung mit einem enzymatischen Verseifungsmittel, insbesondere Esterasen.

Das Reaktionsgemisch aus der Verseifung in Schritt d) wird im nachfolgenden Schritt e) in mindestens einem Trennverfahrensschritt ausgewählt aus der Gruppe, bestehend aus einer thermischen Trennung, beispielsweise Destillation, einer Extraktion, einer Kristallisation oder einer weiteren Membrantrennung, unterworfen, um die in Schritt d) gebildete Carbonsäuresalze vom restlichen Reaktionsgemisch abzutrennen. Das Trennverfahren ist vorzugsweise eine Destillation. Die entsprechenden Verfahrensbedingungen sind dem Fachmann bekannt. Es kann auch eine mehrstufige Destillation durchgeführt werden.

Zudem können in dem mindestens einen Trennverfahrensschritt auch die eingesetzten Alkohole A bzw. B abgetrennt und zur jeweiligen ersten oder zweiten Reaktionszone zurückgeführt werden. Bei der Rückführung kann ein Purgestrom entnommen werde, um beispielsweise Nebenprodukte aus dem Verfahren auszuschleusen.

### Beispiel 1

Umsetzung von Diisobuten (DiB) zu entsprechendem Ester Methyl-3,5,5-trimethylhexanoat (TMH-Ester) und nachfolgender Hydrolyse zu 3,5,5-Trimethylhexansäure (TMH-Säure). Di-iso-buten stellt ein Gemisch bestehend aus den beiden C8-Isomeren 2,4,4-Trimethylpent-1-en und 2,4,4-Trimethylpent-2-en in den Verhältnissen von ungefähr 80:20 dar.

Ein 200 mL Glasautoklav der Firma Büchi wurde verschlossen und abwechselnd (3mal) mit Argon bis zu 10 bar beaufschlagt und wieder entspannt um Sauerstoff zu verdrängen. In einem sekurierten Schlenkkolben wurde unter Argon [Pd(acac)₂] (76,37 mg; 0,06mol% bezogen auf DiB), 1,2-bis((tert-butyl(pyridin-2-yl)phosphanyl)methyl)benzen (218 mg; 0,12mol% bezogen auf DiB), MeOH (84 mL; 500 mol% bezogen auf DiB), H₂SO₄ (147 mg; 0,36mol% bezogen auf DiB), DiB (65,6 mL, 0,41 mol) eingewogen. Mittels Schlauchverbindung und Argonüberdruck wurde die Lösung in den Autoklaven überführt und dieser verschlossen. Nach Ablassen des Argons wurde dieser mit 10 bar CO beaufschlagt. Unter Rühren mit 500 U/min wird Mittels Ölbad (auf Innentemperatur geregelt) der Autoklav auf 120°C aufgeheizt. Nach 10 h wird die Reaktion beendet. Nach Abkühlen und Ablassen des Druckes wurde 1mL Probe gezogen zu der 150 µL Isooctan als interner Standard hinzugefügt, um die Ausbeute und n/iso-Selektivität mittels GC Analyse zu bestimmen. Die Ausbeute betrug 99% für den TMH-Ester (*n*/*iso*: >99). Hochsiedende Nebenprodukte konnten nicht detektiert werden.

Mittels fraktionierter Destillation wurden zunächst Methanol und Spuren nicht umgesetztes Olefin entfernt bevor der TMH-Ester reingewonnen wurde. Für die Hydrolyse wird als Reaktionsgefäß ein verschließbares Druckrohr (pressure tube, ACE Glass Incorporated, 5 mL) verwendet. Der TMH-Ester Methyl-3,5,5-trimethylhexanoat (437,5 mg, 2,54 mmol, 500 µL,) wurde zusammen mit Wasser (2,492 g, 0,138 mol, 2,5 mL) in das Reaktionsgefäß gefüllt. Anschließend werden 75 mg (17,1 wt%) der in Tabelle 1 genannten heterogenen Katalysatoren hinzugefügt. Das Reaktionsgefäß wird verschlossen und in einen vorgeheizten Heizblock gestellt. Die Reaktion wird bei 100 °C unter kontinuierlichem Rühren (550 rpm, Magnetrührer) durchgeführt. Nach 20 Stunden wird die Reaktion beendet, das Reaktionsgefäß aus dem Heizblock genommen und an Luft bis auf Umgebungstemperatur abgekühlt. Anschließend wird eine Probe zur Umsatzkontrolle mittels GC präpariert. Dazu werden 0,1 mL der organischen Phase (Produktgemisch ohne Wasser und Katalysator) zusammen mit 0,05 mL Isooctan als internen Standard und 1 mL Aceton als Lösemittel in einem GC-Vial gemischt. Die Ausbeute (Y) wurde im Anschluss mittels GC-FID ermittelt und ist in Tabelle 7 für den jeweiligen Katalysator notiert.

**Tabelle 7: Vergleich der Ausbeute (TMH-Säure)**

| Katalysator | Ausbeute (TMH-Säure) |
|---|---|
| | % |
| Z-beta-H | 44 |
| Z-beta-H-25 | 43 |
| Z-beta-H-38 | 69 |
| Z-Y-H-60 | 48 |
| Z-Y-H-80 | 50 |
| Z-beta-H-150 | 48 |

## Patentansprüche

1. Verfahren zur Herstellung einer Carbonsäure oder deren Salz, wobei das Verfahren die folgenden Schritte umfasst:
a) Herstellung eines Esters durch Umsetzen (Carbonylieren) eines C2- bis C20-Kohlenwasserstoffs mit mindestens einer Mehrfachbindung, vorzugsweise mit mindestens einer olefinischen Doppelbindung mit Kohlenmonoxid und mit einem Alkohol A in Gegenwart eines homogenen Katalysatorsystems in einer Reaktionszone unter Erhalt eines Produktgemisches;
b) Durchführen einer Membrantrennung zur Abtrennung des homogenen Katalysatorsystems aus dem Produktgemisch, wodurch das homogene Katalysatorsystem und nicht umgesetzter Kohlenwasserstoff und/oder nicht umgesetzter Alkohol A, vorzugsweise nicht umgesetzter Alkohol A im Retentat angereichert werden und der in Schritt a) gebildete Ester im Permeat angereichert wird, wobei als Membranmaterial ein OSN-fähiges (Organic Solvent Nanofiltration) Membranmaterial, welches zumindest eine trennaktive Schicht aufweist, eingesetzt wird, eingesetzt wird;
c) Abtrennen des in Schritt a) gebildeten Esters aus dem Permeat in mindestens einem Trennverfahrensschritt, ausgewählt aus thermischer Trennung, beispielsweise Destillation Extraktion, Kristallisation und Membrantrennung;
d) Hydrolyse oder Verseifen des in Schritt a) hergestellten Esters in Anwesenheit eines sauren Katalysators oder eines Verseifungsmittels unter Erhalt eines Reaktionsgemisches, welches zumindest die Carbonsäure oder deren Salz, den abgespaltenen Alkohol A, Wasser und nicht umgesetzte Ester umfasst;
e) Abtrennen der in Schritt d) gebildeten Carbonsäure oder deren Salz in mindestens einem Trennverfahrensschritt, ausgewählt aus thermischer Trennung, beispielsweise Destillation Extraktion, Kristallisation und Membrantrennung.

2. Verfahren nach Anspruch 1, wobei die trennaktive Schicht aus einem PAEK-Polymer besteht.

3. Verfahren nach Anspruch 2, wobei die trennaktive Schicht aus PEEK besteht, vorzugsweise mit einem Sulfonierungsgrad von weniger als 20%, vorzugsweise weniger als 10%.

4. Verfahren nach einem er vorhergehenden Ansprüche, wobei der Alkohol A ein Mono- oder Polyalkohol (zwei oder mehr OH-Gruppen) mit 1 bis 50 Kohlenstoffatomen, vorzugsweise 1 bis 15 Kohlenstoffatomen, besonders bevorzugt 1 bis 10 Kohlenstoffatomen oder eine Mischung aus zwei oder mehr Mono- und/oder Polyalkoholen ist.

5. Verfahren nach Anspruch 4, wobei der in Schritt a) eingesetzte Alkohol aus der Gruppe, bestehend aus Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol und Mischungen daraus, ausgewählt wird.

6. Verfahren nach einem der vorhergehenden Ansprüche, wobei das bei der Carbonylierung in Schritt a) eingesetzte homogene Katalysatorsystem zumindest ein Metall der Gruppe 8 bis 10 des Periodensystems der Elemente oder einer Verbindung davon, einen vorzugsweise phosphorhaltigen Liganden und eine Säure umfasst.

7. Verfahren nach einem der vorhergehenden Ansprüche, wobei das Kohlenmonoxid, das bei der Umsetzung in Schritt a) eingesetzt wird, dadurch bereitgestellt wird, dass ein Kohlenmonoxid-haltige Gas vorher aufgetrennt wird und das Kohlenmonoxid zur Reaktionszone geleitet wird.

8. Verfahren nach einem der vorhergehenden Ansprüche, wobei das Membranmaterial ein mindestens 300 h in Anwesenheit der Säure des Katalysatorsystems stabiles OSN-fähiges (Organic Solvent Nanofiltration) Membranmaterial ist.

9. Verfahren nach einem der vorhergehenden Ansprüche, wobei die Säure des Katalysatorsystems in Schritt a) eine Brönsted-Säure mit einem pKs ≤ 5, vorzugsweise mit einem pKs ≤ 3 oder eine Lewis-Säure mit einem LAU-Wert von mehr als 25, vorzugsweise mit einem LAU-Wert von 29 ist.

10. Verfahren nach einem der vorhergehenden Ansprüche, wobei die Säure des Katalysatorsystems in Schritt a) eine Brönsted-Säure oder eine Lewis-Säure ist, wobei die Brönsted-Säure Perchlorsäure, Schwefelsäure, Phosphorsäure, Methylphosphonsäure oder eine Sulfonsäure ist und wobei die Lewis-Säure Aluminiumtriflat, Aluminiumchlorid, Aluminiumhydrid, Trimethylaluminium, Tris(pentafluorophenyl)boran, Bortrifluorid, Bortrichlorid oder eine Mischung davon ist.

11. Verfahren nach einem der vorhergehenden Ansprüche, wobei das bei der Membrantrennung in Schritt b) erhaltene Retentat zur Reaktionszone in Schritt a) zurückgeführt und das erhaltene Permeat zum nachfolgenden Schritt c) geführt wird.

12. Verfahren nach einem der vorhergehenden Ansprüche, wobei das Verfahren nach der Abtrennung in Schritt c) die folgenden weiteren Schritte aufweist:
c1) Umestern des in Schritt a) gebildeten Esters mit einem zweiten Alkohol, wobei dieser zweite Alkohol sich von dem im Schritt a) eingesetzten Alkohol unterscheidet, in einer zweiten Reaktionszone unter Erhalt eines zweiten Produktgemischs;
c2) Abtrennen des in Schritt c1) gebildeten Esters und Auftrennung des restlichen zweiten Produktgemisch durch thermische Trennung und/oder mittels Membrantrennung und Rückführung des abgespaltenen ersten Alkohols in die erste Reaktionszone, sowie Rückführung des nicht umgesetzten zweiten Alkohols in die zweite Reaktionszone;
wobei der in Schritt c1) gebildete Ester in der Hydrolyse oder Verseifung in Schritt d) eingesetzt wird.

13. Verfahren nach Anspruch 12, wobei der zweite Alkohol in Schritt e) im Überschuss zugegeben wird.

14. Verfahren nach Anspruch 12 oder 13, wobei der in Schritt e) eingesetzte zweite Alkohol im Vergleich zum ersten Alkohol ein höhersiedender Alkohol ist.

15. Verfahren nach einem der Ansprüche 12 bis 14, wobei ein Teil der abgespaltenen ersten Alkohole bereits aus der zweiten Reaktionszone entnommen und zur ersten Reaktionszone zurückgeführt wird.

## Claims

1. Process for preparing a carboxylic acid or salt thereof, with the process comprising the following steps:
a) preparing an ester by reacting (carbonylating) a C2 to C20 hydrocarbon having at least one multiple bond, preferably having at least one olefinic double bond, with carbon monoxide and with an alcohol A in the presence of a homogeneous catalyst system in a reaction zone to obtain a product mixture;
b) carrying out a membrane separation to separate the homogeneous catalyst system from the product mixture, whereby the homogeneous catalyst system and unreacted hydrocarbon and/or unreacted alcohol A, preferably unreacted alcohol A, are enriched in the retentate and the ester formed in step a) is enriched in the permeate, wherein the membrane material used is used is a membrane material capable of OSN (organic solvent nanofiltration) that includes at least one separation-active layer;
c) separating the ester formed in step a) from the permeate in at least one separation process step selected from thermal separation, for example distillation, extraction, crystallization and membrane separation;
d) hydrolysing or saponifying the ester prepared in step a) in the presence of an acidic catalyst or a saponifying agent to obtain a reaction mixture that comprises at least the carboxylic acid or salt thereof, the eliminated alcohol A, water and unreacted ester;
e) separating the carboxylic acid or salt thereof formed in step d) in at least one separation process step selected from thermal separation, for example distillation, extraction, crystallization and membrane separation

2. Process according to Claim 1, wherein the separation-active layer is composed of a PAEK polymer.

3. Process according to Claim 2, wherein the separation-active layer is composed of PEEK, preferably having a degree of sulfonation of less than 20%, preferably less than 10%.

4. Process according to any of the preceding claims, wherein the alcohol A is a mono- or polyol (two or more OH groups) having 1 to 50 carbon atoms, preferably 1 to 15 carbon atoms, more preferably 1 to 10 carbon atoms, or a mixture of two or more mono- and/or polyols.

5. Process according to Claim 4, wherein the alcohol used in step a) is selected from the group consisting of methanol, ethanol, 1-propanol, 1-butanol, 1-pentanol, 1-hexanol, 2-propanol, tert-butanol, 3-pentanol, 2-propylheptanol, cyclohexanol, phenol or mixtures thereof.

6. Process according to any of the preceding claims, wherein the homogeneous catalyst system used in the carbonylation in step a) comprises at least one metal from groups 8 to 10 of the periodic table of the elements or a compound thereof, a preferably phosphorus-containing ligand and an acid.

7. Process according to any of the preceding claims, wherein the carbon monoxide used in the reaction in step a) is provided by first separating a carbon monoxide-containing gas and passing the carbon monoxide into the reaction zone.

8. Process according to any of the preceding claims, wherein the membrane material is a membrane material capable of OSN (organic solvent nanofiltration) that is stable for at least 300 h in the presence of the acid in the catalyst system.

9. Process according to any of the preceding claims, wherein the acid in the catalyst system in step a) is a Brønsted acid having a pKa ≤ 5, preferably having a pKa ≤ 3, or a Lewis acid having an LAU value of more than 25, preferably having an LAU value of 29.

10. Process according to any of the preceding claims, wherein the acid in the catalyst system in step a) is a Brønsted acid or a Lewis acid, the Brønsted acid being perchloric acid, sulfuric acid, phosphoric acid, methylphosphonic acid or a sulfonic acid and the Lewis acid being aluminium triflate, aluminium chloride, aluminium hydride, trimethylaluminium, tris(pentafluorophenyl)borane, boron trifluoride, boron trichloride or a mixture thereof.

11. Process according to any of the preceding claims, wherein the retentate obtained in the membrane separation in step b) is recycled into the reaction zone in step a) and the permeate obtained is supplied to the subsequent step c).

12. Process according to any of the preceding claims, wherein the process after the separation in step c) includes the following further steps:
c1) transesterifying the ester formed in step a) with a second alcohol, wherein this second alcohol differs from the alcohol used in step a), in a second reaction zone to obtain a second product mixture;
c2) separating the ester formed in step c1) and separating the rest of the second product mixture through thermal separation and/or by means of membrane separation, and recycling the eliminated first alcohol into the first reaction zone and also recycling the unreacted second alcohol into the second reaction zone;
wherein the ester formed in step c1) is used in the hydrolysis or saponification in step d).

13. Process according to Claim 12, wherein the second alcohol in step e) is added in excess.

14. Process according to Claim 12 or 13, wherein the second alcohol used in step e) is a more high-boiling alcohol than the first alcohol.

15. Process according to any of Claims 12 to 14, wherein a proportion of the eliminated first alcohols is already withdrawn from the second reaction zone and recycled into the first reaction zone.

## Revendications

1. Procédé de préparation d'un acide carboxylique ou de son sel, le procédé comprenant les étapes suivantes :
a) préparation d'un ester par transformation (carbonylation) d'un hydrocarbure en C2-C20 présentant au moins une liaison multiple, présentant de préférence au moins une double liaison oléfinique, avec du monoxyde de carbone et avec un alcool A en présence d'un système catalytique homogène dans une zone de réaction avec obtention d'un mélange de produits ;
b) réalisation d'une séparation sur membrane pour séparer le système catalytique homogène à partir du mélange de produits, suite à quoi le système catalytique homogène et l'hydrocarbure non transformé et/ou l'alcool A non transformé, de préférence l'alcool A non transformé, sont enrichis dans le rétentat et l'ester formé dans l'étape a) est enrichi dans le perméat, un matériau membranaire apte à l'OSN (Organic Solvent Nanofiltration - nanofiltration de solvant organique) qui présente au moins une couche active en séparation étant utilisé étant utilisé comme matériau membranaire ;
c) séparation de l'ester formé dans l'étape a) à partir du perméat dans au moins une étape de procédé de séparation, choisie parmi la séparation thermique, par exemple la distillation, l'extraction, la cristallisation et la séparation sur membrane ;
d) hydrolyse ou saponification de l'ester préparé dans l'étape a) en présence d'un catalyseur acide ou d'un agent de saponification avec obtention d'un mélange réactionnel qui comprend au moins l'acide carboxylique ou son sel, l'alcool A dissocié, de l'eau et l'ester non transformé ;
e) séparation de l'acide carboxylique ou de son sel formé dans l'étape d) dans au moins une étape de procédé de séparation, choisie parmi la séparation thermique, par exemple la distillation, l'extraction, la cristallisation et la séparation sur membrane.

2. Procédé selon la revendication 1, la couche active en séparation étant constituée par un polymère de type PAEK.

3. Procédé selon la revendication 2, la couche active en séparation étant constituée par de la PEEK, présentant de préférence un degré de sulfonation inférieur à 20%, de préférence inférieur à 10%.

4. Procédé selon l'une quelconque des revendications précédentes, l'alcool A étant un monoalcool ou un polyalcool (deux groupes OH ou plus) comprenant 1 à 50 atomes de carbone, de préférence 1 à 15 atomes de carbone, de manière particulièrement préférée 1 à 10 atomes de carbone ou un mélange de deux monoalcools et/ou polyalcools ou plus.

5. Procédé selon la revendication 4, l'alcool utilisé dans l'étape a) étant choisi dans le groupe constitué par le méthanol, l'éthanol, le 1-propanol, le 1-butanol, le 1-pentanol, le 1-hexanol, le 2-propanol, le tert-butanol, le 3-pentanol, le 2-propylheptanol, le cyclohexanol, le phénol et leurs mélanges.

6. Procédé selon l'une quelconque des revendications précédentes, le système catalytique homogène utilisé lors de la carbonylation dans l'étape a) comprenant au moins un métal des groupes 8 à 10 du système périodique des éléments ou un composé correspondant, un ligand contenant de préférence du phosphore et un acide.

7. Procédé selon l'une quelconque des revendications précédentes, le monoxyde de carbone, qui est utilisé lors de la transformation dans l'étape a), étant préparé en ce qu'un gaz contenant du monoxyde de carbone est séparé au préalable et le monoxyde de carbone est guidé vers la zone de réaction.

8. Procédé selon l'une quelconque des revendications précédentes, le matériau membranaire étant un matériau membranaire apte à l'OSN (nanofiltration de solvant organique) stable pendant au moins 300 h en présence de l'acide du système catalytique.

9. Procédé selon l'une quelconque des revendications précédentes, l'acide du système catalytique dans l'étape a) étant un acide de Brönsted présentant un pKa ≤ 5, de préférence un pKa ≤ 3 ou un acide de Lewis présentant une valeur LAU supérieure à 25, de préférence une valeur LAU de 29.

10. Procédé selon l'une quelconque des revendications précédentes, l'acide du système catalytique dans l'étape a) étant un acide de Brönsted ou un acide de Lewis, l'acide de Brönstedétant l'acide perchlorique, l'acide sulfurique, l'acide phosphorique, l'acide méthylphosphonique ou un acide sulfonique et l'acide de Lewis étant le triflate d'aluminium, le chlorure d'aluminium, l'hydrure d'aluminium, le triméthylaluminium, le tris(pentafluorophényl)borane, le trifluorure de bore, le trichlorure de bore ou un mélange de ceux-ci.

11. Procédé selon l'une quelconque des revendications précédentes, le rétentat obtenu lors de la séparation membranaire dans l'étape b) étant recyclé dans la zone de réaction dans l'étape a) et le perméat obtenu étant guidé vers l'étape suivante c).

12. Procédé selon l'une quelconque des revendications précédentes, le procédé présentant les autres étapes suivantes après la séparation dans l'étape c) :
c1) transestérification de l'ester formé dans l'étape a) avec un deuxième alcool, ce deuxième alcool se distinguant de l'alcool utilisé dans l'étape a), dans une deuxième zone de réaction avec obtention d'un deuxième mélange de produits ;
c2) séparation de l'ester formé dans l'étape c1) et séparation du deuxième mélange résiduel de produits par séparation thermique et/ou au moyen d'une séparation membranaire et recyclage du premier alcool dissocié dans la première zone de réaction ainsi que recyclage du deuxième alcool non transformé dans la deuxième zone de réaction ;
l'ester formé dans l'étape c1) étant utilisé dans l'hydrolyse ou la saponification dans l'étape d).

13. Procédé selon la revendication 12, le deuxième alcool dans l'étape e) étant ajouté en excès.

14. Procédé selon la revendication 12 ou 13, le deuxième alcool utilisé dans l'étape e) étant un alcool à point d'ébullition plus élevé que celui du premier alcool.

15. Procédé selon l'une quelconque des revendications 12 à 14, une partie des premiers alcools dissociés étant déjà prélevée de la deuxième zone de réaction et recyclée dans la première zone de réaction.
